# EUROPÄISCHE PATENTSCHRIFT

(11) **EP 1 194 133 B1**
(45) Veröffentlichungstag und Bekanntmachung des Hinweises auf die Patenterteilung: **02.11.2005**
(21) Anmeldenummer: 00947871.0
(22) Anmeldetag: 23.06.2000
(51) Int. Cl.: A61K 9/70

(54) **WIRKSTOFFHALTIGEN MEHRSCHICHTFILM AUS IN SITU VERNETZTEN HYDROPHILEN POLYMEREN**
MULTILAYERED FILM CONTAINING AN ACTIVE SUBSTANCE AND MADE OF CROSS-LINKED HYDROPHILIC POLYMERS
FILM MULTICOUCHE RENFERMANT UN PRINCIPE ACTIF ET CONSTITUE DE POLYMERES HYDROPHILES RETICULES IN SITU

(30) Priorität: 13.07.1999 DE 19932603
(43) Veröffentlichungstag der Anmeldung: 10.04.2002
(73) Patentinhaber: Grünenthal GmbH, 52078 Aachen (DE)
(72) Erfinder: RUPPRECHT, Herbert, D-93053 Regensburg (DE); ZINZEN, Stephan, D-22299 Hamburg (DE)
(86) Internationale Anmeldenummer: PCT/EP2000/005824
(87) Internationale Veröffentlichungsnummer: WO 2001/003917

(56) Entgegenhaltungen:
- US-A- 5 641 504
- RUPPRECHT H; ZINZEN S; ABLETSHAUSER C: "Drug release regulation by in situ crosslinked films based on hydrophilic polymers" FARMACEVTSKI VESTNIK, Bd. 48, 1997, Seiten 220-221, XP000981211
- ABLETSHAUSER C B ET AL: "FILM COATING OF PELLETS WITH INSOLUBLE POLYMERS OBTAINED IN SITU CROSSLINKING IN THE FLUIDIZED BED" JOURNAL OF CONTROLLED RELEASE,NL,ELSEVIER SCIENCE PUBLISHERS B.V. AMSTERDAM, Bd. 27, Nr. 2, 1. November 1993 (1993-11-01), Seiten 149-156, XP000400403 ISSN: 0168-3659

## Beschreibung

Die vorliegende Erfindung betrifft einen wirkstoffhaltigen Mehrschichtfilm aus filmbildenden Polymeren mit einer Deckschicht, mindestens einer wirkstoffhaltigen Schicht und einer Haftschicht, ein Verfahren zu dessen Herstellung, sowie dessen Verwendung.

Die Abgabe von Wirkstoffen aus wirkstoffhaltigen Mehrschichtfilm gewinnt heutzutage zunehmend an Bedeutung. Diese Mehrschichtfilme bestehen aus einer Deckschicht, die den Mehrschichtfilm u. a. vor unerwünschten Einflüssen schützt, einer wirkstoffhaltigen Schicht und einer Haftschicht, mit der der Mehrschichtfilm an dem Ort, an dem der Wirkstoff abgegeben werden soll, befestigt wird.

Insbesondere die Verabreichung von Arzneistoffen über transmucosale Applikation mittels eines Mehrschichtfilms hat sich sowohl für die lokale als auch für die systemische Therapie als sehr vorteilhaft erwiesen.

Die Herstellung von Filmen, welche die Anforderungen zur Applikation in der Mundhöhle hinsichtlich Dicke, Flexibilität, Mucoadhäsion, Steuerung der Wirkstoffliberation und Verträglichkeit erfüllen, stößt jedoch bisher auf große technologische Schwierigkeiten. So muß zur Aufrechterhaltung der Langzeitwirkung, insbesondere aber für die Deckschicht eine Unlöslichkeit im wässrigen Milieu des Applikationsortes gefordert werden. Dies bedingt den Einsatz von wasserunlöslichen Polymeren wie z. B. Ethylcellulose, Polymethylmethacrylaten u. ä.; d. h. Substanzen, die sich nur in organischen Lösungsmitteln oder in Form von komplizierten Latex- oder Pseudolatexdispersionen entsprechend verarbeiten lassen, wodurch sich sowohl ökologische als auch ökonomische Nachteile ergeben. Da diese Mehrschichtfilme i.d.R. durch ein Gießverfahren hergestellt werden, ergeben sich darüber hinaus auch erhebliche Probleme bei der Übertragung des Herstellungsverfahrens aus dem Labor- in den Produktionsmaßstab.

Es stellt sich deshalb die Aufgabe, einen Mehrschichtfilm, ein Verfahren und eine Vorrichtung zu dessen Herstellung zur Verfügung zu stellen, die die Nachteile des Standes der Technik nicht aufweisen.

Die Aufgabe wird erfindungsgemäß durch die Bereitstellung eines Mehrschichtfilms gemäß Anspruch 1 gelöst.

In situ vernetzt im Sinne der Erfindung bedeutet, daß das filmbildende Polymer und ein geeignetes Vernetzungsmittel getrennt voneinander auf eine vorzugsweise glatte Oberfläche aufgebracht werden und sich dort vermischen, wobei es nach deren Vermischung zu einer in situ Vemetzung kommt. In situ vernetzte Filme werden z.B. von Rupprecht H., Zinzen S. und Abletshauser C. beschrieben in "Drug release regulation by in situ crosslinked films based on hydrophilic polymers" ; Farmacevtski Vestnik, Bd. 48, 1997, Seiten 220-221.

Die Deckschicht dient zur mechanischen Stabilisierung des Mehrschichtfilms, zum Schutz des in der wirkstoffhaltigen Schicht eingeschlossenen Wirkstoffes sowie als Barriere gegen die Wirkstoffdiffusion, so daß eine unidirektionale Freisetzung des Wirkstoffes durch die Haftschicht zu dem Ort erfolgt, an dem der Mehrschichtfilm angeklebt ist.

Die Deckschicht kann aus einem beliebigen filmbildenden, wasserlöslichen Polymeren und geeigneten Vernetzungsmitteln hergestellt werden, wie sie z.B. bei Abletshauser, C.; Schneider, R.; Rupprecht, H.: Film coating of pellets with insoluble polymers obtained by "in situ" crosslinking in the fluidized bed. J. Control. Rel 27, 149 - 156 (1993) oder bei Abletshauser, C.; Rupprecht, H.: Self supporting polymer films crosslinked "in situ" by simultaneous spraying of component solutions. Farm. Vestn. 45, 297 - 309 (1994) beschrieben sind, die hiermit als Referenz eingeführt werden und somit Teil der Offenbarung sind.

Vorzugsweise basiert die Deckschicht jedoch auf Celluloseether, vorzugsweise Hydroxyethylcellulose und/oder Methylcellulose, besonders bevorzugt Methylhydroxypropylcellulose, wobei die Celluloseether vorzugsweise mit phenolischen Substanzen, besonders bevorzugt mit Tannin vernetzt sind.
Zur Optimierung der Filmeigenschaften kann das Mengenverhältnis Polymer/Vernetzungsmittel in einem breiten Bereich variiert werden. Vorzugsweise beträgt das Mengenverhältnis jedoch 4:1 bis 1:1.

Ganz besonders bevorzugt basiert die Deckschicht aus mit Tannin vernetzter Methylhydroxypropylcellulose, wobei das Vernetzungsverhältnis zwischen 4:1 und 1:2 beträgt.

Ebenfalls bevorzugt basiert die Deckschicht auf anionischen Polymeren, vorzugsweise Natriumcaboxymethylcellulose, Polyacrylate oder Carragenate, besonders bevorzugt Natriumalginat, wobei die anionischen Polymere vorzugsweise mit anorganischen Ionen, besonders bevorzugt Kalciumionen, oder vorzugsweise mit Polykationen, besonders bevorzugt Chitosan vernetzt sind.

Weitere bevorzugte Deckschichtmaterialien sind Filme aus Mischungen von Tannin vernetzter Methylhydroxypropylcellulose mit Latexdispersionen von Ethylcellulose, geeigneten Polymethylmethacrylate, wie z. B. Eudragit RS, Eudragit E ect., von Röhm Pharma GmbH, Darmstadt.

Als Deckschichten kommen außerdem besonders bevorzugt vorgefertigte Folien aus wasserundurchlässigen, oberflächig hydrophilen Materialien wie Polyamiden in Frage.

Die Deckschicht kann auch aus einer Mischung von Polymeren hergestellt werden, um z.B. die Eigenschaften dieser Schicht zu optimieren.

Die Funktion der Deckschicht läßt sich erfindungsgemäß weiter durch Einbringen von Farbstoffpigmenten wie z. B. Eisenoxide, Titandioxid ect. und/oder durch Süß- und / oder Aromastoffen sowie ggf. durch weitere Hilfsstoffe zur Optimierung der Gebrauchseigenschaften, vorzugsweise als Arzneimittelform erweitern.

Vorzugsweise beträgt die Schichtdicke der Deckschicht zwischen 30 und 100 µm, besonders bevorzugt zwischen 40 und 60 µm.

Der Mehrschichtfilm weist erfindungsgemäß mindestens eine wirkstoffhaltige Schicht auf, die sich unmittelbar an die Deckschicht anschließt. Diese Schicht dient erfindungsgemäß als Wirkstoffmatrix.

Die wirkstoffhaltige Schicht kann aus einem beliebigen filmbildenden, wasserlöslichen Polymeren und geeigneten Vernetzungsmitteln hergestellt werden, wie sie z.B. bei Abletshauser, C.; Schneider, R.; Rupprecht, H.: Film coating of pellets with insoluble polymers obtained by "in situ" crosslinking in the fluidized bed. J. Control. Rel 27, 149 - 156 (1993) oder bei Abletshauser, C.; Rupprecht, H.: Self supporting polymer films crosslinked "in situ" by simultaneous spraying of component solutions. Farm. Vestn. 45, 297 - 309 (1994) beschrieben sind, die hiermit als Referenz eingeführt werden und somit Teil der Offenbarung sind.

Vorzugsweise basiert die wirkstoffhaltige Schicht jedoch auf Celluloseether, vorzugsweise Hydroxyethylcellulose und/oder Methylcellulose, besonders bevorzugt Methylhydroxypropylcellulose, wobei die Celluloseether vorzugsweise mit phenolischen Substanzen, besonders bevorzugt mit Tannin vernetzt sind.

Zur Optimierung der Filmeigenschaften kann das Mengenverhältnis Polymer/Vernetzungsmittel in einem breiten Bereich variiert werden. Vorzugsweise beträgt das Mengenverhältnis jedoch 4:1 bis 1:1.

Ebenfalls bevorzugt basiert die wirkstoffhaltige Schicht auf anionischen Polymeren, vorzugsweise Natriumcaboxymethylcellulose, Polyacrylate oder Carragenate, besonders bevorzugt Natriumalginat, wobei die anionischen Polymere vorzugsweise mit anorganischen Ionen, besonders bevorzugt Kalciumionen oder vorzugsweise mit Polykationen, besonders bevorzugt Chitosan vernetzt sind.

Die wirkstoffhaltige Schicht kann auch aus einer Mischung von Polymeren hergestellt werden, um z.B. die Wirkstofffreisetzung aus dieser Schicht und/oder deren Stabilität zu optimieren. Vorzugsweise basiert die wirkstoffhaltige Schicht auf mit Tannin vernetzter Methylhydroxypropylcellulose, der zur Steuerung der Wirkstofffreisetzung und zur besseren Haftung an der Deck- bzw. Haftschicht weitere Polymere, vorzugsweise mit Kalciumionen vernetztes Alginat, besonders bevorzugt Polyacrylsäure (z.B. Carpol 934 P der Firma BF Goodrich, Cleveland, Ohio, USA) in Mengen von 5-50 Gew.-% bezogen auf das Polymer der wirkstoffhaltigen Schicht, z. B. der Methylhydroxypropylcellulose, zugesetzt wird.

Der erfindungsgemäße Mehrschichtfilm weist eine oder mehrere wirkstoffhaltige Schichten auf. Diese Schichten enthalten vorzugsweise denselben Wirkstoff, wobei jede Schicht jeweils über ein anderes Freisetzungsprofil verfügt. Ebenfalls bevorzugt weisen diese Schichten jeweils mehrere Wirkstoffe auf, wobei die Schichten darüber hinaus vorzugsweise jeweils über unterschiedliche Freisetzungsprofile verfügen.

Die wirkstoffhaltige Schicht weist bezüglich des jeweiligen Wirkstoffes horizontale und/oder vertikale Gradienten auf, oder der jeweilige Wirkstoff ist in bestimmten horizontalen und/oder vertikalen Segmenten der wirkstoffhaltigen Schicht konzentriert.

Um ein gewisses Freigabeprofil zu erreichen, kann es z.B. wünschenswert sein wirkstofffreie Teilschichten zu haben.

Die Dicke der wirkstoffhaltigen Schicht wird erfindungsgemäß der zu inkorporierenden Wirkstoffmenge angepaßt. Vorzugsweise beträgt die Schichtdicke der wirkstoffhaltigen Schichten jeweils zwischen 30 und 100 µm, besonders bevorzugt zwischen 40 und 60 µm.

Bezüglich der in der wirkstoffhaltigen Schicht enthaltenen Wirkstoffe gibt es prinzipiell keine Einschränkung. Vorzugsweise sind die Wirkstoffe jedoch Duftstoffe, Aromen, Diagnostica, Pflanzenschutzmittel, pharmazeutische Wirkstoffe, Vitamine, Nährstoffe und/oder Düngemittel. Als pharmazeutische Wirkstoffe können Analgetica, Antiallergica, Antibiotica, Antiemetica, Antiseptica, Antihistaminica, Antihypertonia, Appetitzügler, Cardiaca, Chemotherapeutica, Enzympräparate, Hormone, Immunmodulatoren, Lokalanästhetica, Psychopharmaka, Spasmolytica, Virustatica, Vitamine und Zytostatica verwendet werden.

Geeignete Wirkstoffe sind, Diamorphin, Alflentanil, Sufentanyl, Pentazocin, Buprenorphin, Nefopam, Flupirtin, Tramadol, Oxycodon, Metamizol, Propyphenanzon Phenazone, Nifenazon, Phenylbutazon, Oxyphenbutazon, Mofebutazon, Diflunisal, Meptazinol, Methadon, Pethidin, Meloxicam, Fenbufen, Mefenamisäure, Tenoxicam, Azapropazon, Piritramid, Tramadol, Amantadin, Benzotropin, Procyclidin, Moclobemid, Tranylcypromid, Maprotilin, Doxepin, Opipramol, Desipramin, Imipramin, Fluroxamin, Paroxetin, Trazodon, Viloxazin, Fluphenazin, Perphenazin, Promethazin, Thioridazin, Triflupromazin, Prothipendyl, Tiotixen, Chlorprothixen, Pipamperon, Pimozid, Fenethyllin, Trifluoperazin, Thioridazin, Oxazepam, Alprazolam, Clobazam, Piracetam, Melfalan, Cyclophosphamid, Trofosfamid, Chlorambucil, Lomustin, Busilfan, Prednimustin, Mercaptopurin, Thioguanin, Hydroxycarbamid, Altretamin, Procarbazin, Lisurid, Methysergid, Pizotifen, Roxatidin, Pirenzipin, Proglumid, Bromoprid, Pheniramin, Dimethinden, Tritoqualin, Loratadin, Doxylamin, Mequitazin, Dexchlorpheniramin, Triprolidin, Oxatomid, Moxonidin, Doxazosin, Urapidil, Dihydralazin, Deserpidin, Alprenolol, Bupranolol, Penbutolol, Esmolol, Ciliprolol, Metipranolol, Nadolol, Quinapril, Fosinopril, Cilazapril, Democlocyclin, Lymecyclin, Oxytetracyclin, Sulfamethopyrazin, Aerosoxacin, Becampicillin, Piperacillin, Pivampicillin, Cloxacillin, Flucloxacillin, Metronidazol, Clindamycin, Cefaclor, Cefpodoxim, Cephalexin, Cefradin, Pirbuterol, Orciprenalin, Clenbuterol, Procaterol, Cholintheophyllinat, Theophyllinethylenediamin, Ketofen, Viquidil, Procainamid, Mexiletin, Tocainid, Ipratropium, Tobutamid, Gliquidon, Gliborurid, Tolazamid, Acarbose und pharmazeutisch aktive Salze oder Ester der vorgenannten Wirkstoffe sowie Kombinationen von zwei oder mehreren dieser Wirkstoffe oder deren Salze oder Ester.

Geeignete Wirkstoffe sind beispielsweise Acebutolol, Acetylcystein, Acetylsalicylsäure, Aciclovir, Albrazolam, Alfacalcidol, Allantoin, Allopurinol, Ambroxiol, Amikacin, Amiloride, Aminoessigsäure, Amiodaron, Amitriptylin, Amlodipin, Amoxicillin, Ampicillin, Ascorbic Acid, Aspartam, Astemizole, Atenolol, Beclometason, Benserazid, Benzalkonium Hydrodrochlorid, Benzocain, Benzoesäure, Betametasone, Bezafibrate, Biotin, Biperiden, Bisoprolol, Bromacepam, Bromhexine, Bromocriptine, Budesonide, Bufexamac, Buflomedil, Buspirone, Coffein, Campher, Captopril, Carbamacipine, Carbidopa, Carboplatin, Cefaclor, Cefalexin, Cefadroxil, Cefazolin, Cefixime, Cefotaxim, Ceftazidin, Ceftriaxon, Cefuroxim, Celedilin, Chloramhenicol, Chlorhexidin, Chlorpheniramin, Chlortalidon, Cholin, Ciclosporin, Cilastatin, Cimetidin, Ciprofloxacin, Cisaprid, Cisplatin, Clarithromycin, Clavulansäure, Clomibramin, Clonazepam, Clonidin Clotrimazol, Codein, Colestyramin, Cromoglicinsäure, Cyanocobalamin, Cyproteron, Desogetrel, Dexamethason, Dexpanthenol, Dexthromethorphan, Dextropropoxiphen, Diazepam, Diclofenac, Digoxin, Dihydrocodein, Dihyderoergotamin, Dihydroergotoxin, Diltiazem, Diphenhydramin, Dipyridamol, Dipyron, Disopyramid, Domperidon, Dopamin, Doxcyclin, Enalapril, Ephedrin, Epinephrine, Ergocalciferol, Ergotamin, Erytrhomycin, Estradiol, Ethinylestradinol, Etoposid, Famotidin, Felodipin, Fenofibrat, Fenoterol, Fentanyl, Flavin Mononucleotid, Fluconazol, Flunarizin, Fluorouracil, Fluoxetin, Flurbiprofen, Furosemid, Gallopamil, Gemfibrozil, Gentaminicin, Ginkgo Biloba, Glibenclamid, Glipizid, Glozapin, Glycyrrhiza Glabra, Griseofulvin, Guaifenesin, Haloperidol, Heparin, Hyaluronsäure, Hydrochlorothiazid, Hydrocodon, Hydrocortison, Hydromorphon, Ibratropium Hydroxide, Ibuprofen, Imipenem, Indomethacin, lohexol, Iopamidol, Isosorbid Dinitrat, Isosorbid Mononitrat, Isotretionin, Ketotifen, Ketoconazol, Ketoprofen, Ketorolac, Labatalon, Lactulose, Lecithin, Levocarnitin, Levodopa, Levoglutamid, Levonorgestrel, Levothyroxin, Lidocain, Lipase, Lipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Monoxidil, Misoprostol, Morphin, Multivitamine und Mineralien, N-Methylephedirn, Naftidrofuril, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Phenoxifyllin, Phenoxymethylpenicillin, Phenylephrin, Phenylpropanolamin, Phenytoin Piroxicam, Polymxyxin B, Povidone-Iod, Pravastatin, Prazepam, Prazosin, Prednisolon, Prednison, Propafenon, Propranolol, Proxyphyllin, Pseudoephedrin, Pyridoxine, Chinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolone Acetonide, Triamteren, Trimethoprim, Troxerutin, Uracil, Valproinsäure, Vancomycin, Verapamil, Vitamine E, Zidovudine.

Weiterhin geeignete Wirkstoffe, die von der erfindungsgemäßen Vorrichtung abgegeben werden können, sind Proclorperazin-edisylal, Eisen-II-sulfat, Aminocapronsäure, Kaliumchlorid, Mecamylamin-hydrochlorid, Procainamidhydrochlorid, Amphetamin-sulfat, Benzphetamin-hydrochlorid, Isoporterenolsulfat, Methamphetamin-hydrochlorid, Phenmetrazin-hydrochlorid, Bethanecholchlorid, Methacholin-chlorid, Pilocarpin-hydrochlorid, Atropinsulfat, Methascopolamin-bromid, Isopropamid-iodid, Tridihexethylchlorid, Phenformin-hydrochlorid, Methylphenidat-hydrochlorid, Oxprenolol-hydrochlorid, Metroprolol-tartrat, Cimetidin-hydrochlorid, Diphenidol, Meclizin-hydrochlorid, Proclorperazinmaleat, Phenoxybenzamin, Thiethylperazin-maleat, Anisindon, Diphenadion, Erythrit-tetranitrat, Dizoxin, Isofurophat, Acetazolamid, Methazolamid, Bendroflumethiazid, Chlorpropamid, Tolazamid, Chlormadinon-acetat, Phenaglycodol, Aluminium-aspirin, Methotrexat, Acetyl-sulfioxazol, , Progestine, österrogene Steroide, progestatine Steroide, Corticosteroide, 17-β-östradiol, Ethinylöstradiol-3-methyl-ester, Hydrocorticosteron-acetat, Methyltesteron, 17-α-Hydroxyprogesteron-acetat, 19-Nor-progesteron, Norethindron, Progesteron, Norgesteron, Norethynodrel u. ä.

Beispiele für andere Wirkstoffe, die mit Hilfe der Vorrichtung abgegeben werden können, sind, Fenoprofen, Sulindac, Indoprofen, Nitroglycerin, Timolol, Alprenolol, Imipramin, Chlorpromazin, Dihydroxyphenylalanin, Pivaloxyloxyethylester von α-Methyldopa-hydrochlorid, Calcium-gluconat, Eisen-II-lactat, Vincamin, Phenoxybenzamin, Blocker u. ä. Die Wirkstoffe sind bekannt aus "Pharmaceutical Sciences" von Remington, 14. Auflage, 1979, Mack Publishing Co., Easton, Pennsylvania; "The Drug, The Nurse, The Patient, Including Current Drug Handbook", 1974-1976, von Falconer et al, Saunder Co., Philadelphia, Pennsylvania, und "Medical Chemistry", 3. Auflage, Band 1 und 2, von Burger, Wiley-Interscience, New York.

Repräsentative Arzneimittel, die an warmblütige Tiere, beispielsweise Wiederkäuer, mit Hilfe des erfindungsgemäßen Abgabesystems verabreicht werden können, sind u. a. Anthelmintica, wie Mebendazol, Levamisol, Albendazol, Cambendazol, Fenbendazol, Parbendazol, Oxfendazol, Oxybendazol, Thiabendazol, Tichlorfon, Praziquantel, Morantel und Pirantel, u. ä.; Antiparasiten-Mittel, wie Avermectine und Ivermectin, wie in den US-PS 41 99 569 und 43 89 397 (Merck) und in "Science", Band 221, S. 823 - 828, 1983 angegeben, wo diese Ivermectin-Antiparasiten-Mittel als geeignet zur Unterstützung bei der Bekämpfung von üblicherweise bei Säugetieren auftretenden Würmern, wie Rundwürmern (Spulwürmern), Lungenwürmern u. ä., angegeben sind, und auch daß das Ivermectin geeignet ist zur Behandlung von Insekteninfektionen, wie Maden, Läusen, Mübenräude u. ä.; antimikrobielle Mittel, wie Chlortetracyclin, Oxytetracyclin, Tetracyclin, Gentamicin, Streptomycin, Dihydrostreptomycin, Bacitracine, Erthromycin, Ampicilline, Penicilline, Cephalosporine u. ä.; Schwefel enthaltende Arzneimittel (sufa drugs), wie Sulfamethazin, Sulfathiazol u. ä.; Wachstumsstimulantien, wie Monesin®-natrium und Elfazepam®; Anti-Flohmittel (defleaing agents), wie Dexamethazon und Flumethazon; die Verdauung im Pansen beeinflussende Mittel und Ionophore, wie Lasalocid, Virginamiycin, Salinomycin und Ronnel; Mineralstoffe, wie Kupferoxid, Cobaltsulfat, Kaliumiodat, Zinkoxid, Mangansulfat, Zinksulfat, Selen, Natriumselenit, günstige Mineralsalze u. ä.; Antiblähmittel, wie organische Polysiloxane; hormonelle Wachstumszusätze, wie Stilböstrol; Vitamine, wie Vitamine A und D; mit 500 000 : 100 000 IU/f, Vitamin E mit 500 000 IU/f u. ä.; Antienteritismittel, wie Furazolidon, Wachstumsfaktoren, Nährstoffzusätze, wie Lysinmonohydrochlorid, Methionin, Magnesiumcarbonat u. ä.; β-Agonisten, Elenbuterol u. ä., und chemische Markierungsstoffe, wie Chromoxid, und Salze von Ytterbium und Erbium.

Die lokal wirkenden pharmazeutischen Wirkstoffe umfassen weiterhin Fungizide wie Amphotericin B, Antibiotika, wie Penicilline, Cephalosporine, Erythromycin, Tetracyclin, Aminoglycoside, antivirale Verbindungen wie Acyclovir, Idoxuridin, Atemverbesser wie Chlorophyll; Gewebewachstums hemmende Verbindungen, Antikariesverbindungen wie Metallfluoride, insbesondere Natriummonofluorphosphat, Zinnfluorid, Aminfluorid, Schmerzmittel wie Methylsalicylat, Lokalanästhetika wie Benzocain, orale Antiseptika wie Chlorhexidin und dessen Salze, Hexylresorcin, Dequalinium Chlorid, Cetylpyridin Chlorid), Antientzündungsmittel, Hormone wie Oestriol, Antiplaqueverbindungen wie Chlorhexidin und dessen Salze, Octenidin, oder Mischungen von Thymol, Menthol, Methysalicylat, Eucalyptol, Pufferverbindungen wie Caliumphosphat, Calciumcarbonat, Natriumbicarbonat, Natrium- und Caliumhydroxid sowie Desensibilisatoren für Zähne wie z. B. Caliumnitrat.

Desweiteren sind als aktive Wirkstoffe Desinfektiva wie Chlorverbindungen, insbesondere Calciumhypochlorit, ein Insektizid, Pestizid, Herbizid, Fungizid, oder Wachstumförderer bzw. Düngemittel wie z. B. Stickstoff haltige Verbindungen, insbesondere Harnstoff, Harnstofformaldehydverbindungen, Caliumnitrat, Caliumsulfat, Caliumchlorid, Ammoniumnitrat, Ammoniumsuflat, Monoammoniumphosphat, dibasisches Ammoniumphosphat, Ammoniumphosphorsäureverbindungen, Spurenelemente für Nahrungsmittel wie Eisen, Zink, Mangan, Kupfer, Bor, Molybden oder Mischungen daraus

Wirkstoffe, die sich zur Herstellung der erfindungsgemäß transdermalen Systeme eignen sich auch Steroidhormone wie:
Gestagen wirksame Steroidhormone, wie beispielsweise das 13-Ethyl-17β-hydroxy-18,19-dinor-17α-pregn-4-en-20yl-3-on, das 13-Ethyl-17β-hydroxy-18,19-dinor-17α-pregna-4,15-dien-20yn-3-on (=Gestoden), das 13-Ethyl-17β-hydroxy-11-methylen-18,19-dinor-17α-pregn-4-en-20yn oder das 13-Ethyl-11-methylen-17β-hydroxy-18,19-dinor-17α-pregn-4-en-3-on (3-Keto-Desogestrel) Estrogen wirksame Steroidhormone das 3-Hydroxy-1,3,5-(10)-estratrien-17-on (=Estron), das 1,3,5(10)-Estratrien-3,17β-diol oder das 1,9-Nor-17α-pregna-1,3,5(10)-trien-20yn-3,17β-diol, das 17β-Hydroxy-19-nor-17α-pregn-4en-20yn-3-on, das 14α, 17α-Ethano-1,3,5(10)-estratrien-3,17β-diol (=Cyclodiol) und das 14α, 17α-Ethano-1,3,5(10)-estratrien-3,16α,17β-triol (=Cyclotriol) und Kombinationen dieser Gestagene und Estrogene.
Androgen wirksame Steroidhormone wie das 17β-Hydroxy-4-androsten-3-on (=Testosteron) und dessen Ester oder das 17β-Hydroxy-1α-methyl-5α-androsten-3-on (=Mesterolon).
Antiandrogen wirksame Steroidhormone wie das 17α-Acetoxy-6-chlor-1β,2β-dihydro-3H-cyclopropa[1,2]-pregna-1,4,6-trien-3,20-dion.
Kortikoide wie das 11β,17α,21-Trihydroxy-4-pregnen-3,20-dion, das 11β,17α,21-Trihydroxy-1,4-pegnadien-3,20-dion, das 11β,17α,21-Trihydroxy-6α-methyl-1,4-pregnatrien-3,20-dion und das 6α-Fluor-11β,21-dihydroxy-16α-methyl-1,4-pregnadien-3,20-dion (=Diflucortolon) und deren Ester.

Geeignete Wirkstoffe sind ferner:
Ergolin-Derivate, wie das Lisurid, [3-(9,10-Didehydro-6-methyl-8α-ergolinyl)-1,1-diethylharnstoff], das Bromlisurid [=3-(2-Brom-9,10-dehydro-6-methyl-8α-ergolinyl-1,1-diethylharnstoff], das Tergurid [=3-(6-Methyl-8α-ergolinyl-1,1-diethylharnstoff] und das Protergurid [=3-(6-Propyl-8α-ergolinyl)-1,1-diethylharnstoff].
Antihypertonika wie das 7α-Acetylthio-17α-hydroxy-3-oxo-4-pregnen-21-carbonsäure-γ-lacton und das 7α-Acetylthio-15β-,16β-methylen-3-oxo-17α-pregna-1,4-dien-21,17-carbolacton (=Mespirenon).
Antikoagulantia wie die 5-[Hexahydro-5-hydroxy-4-(3-hydroxy-4-methyl-1-octen-6-ynyl)-2(1H)-pentalenyliden)]-pentansäure (=Iloprost) oder die (Z)-7-[(1R,2R,3R,5R)-5-Chlor-3-hydroxy-2-[(E)-(3R)-3-hydroxy-4,4-dimethyl-1-octenyl]-cyclopentyl]-5-heptensäure (=Nocloprost).
Psychopharmaka wie das 4-(3-Cyclopentyloxy-4-methoxy-phenyl-2-pyrrolidon (=Rolipram) und das 7-Chlor-1,3-dihydro-1-methyl-5-phenyl-2H-1,4-benzodiazepin-2-on.

Bezogen auf die Filmmasse weist der erfindungsgemäße Mehrschichtfilm vorzugsweise bis zu 30% Wirkstoff auf, ohne daß sich die mechanischen Eigenschaften des Mehrschichtfilms verändern.

Die zum Applikationsort gerichtete Haftschicht wird unmittelbar auf den wirkstoffhaltigen Schichten angebracht. Sie besteht erfindungsgemäß im wesentlichen aus nicht vernetzten, vorzugsweise mucoadhäsiven Polymeren, besonders bevorzugt aus Polyacrylsäure (Carbopol 934 P, ca. 80 %) und Methylcellulose (Metholose 90 SH 100, ca. 20 %) mit einer Filmdicke von vorzugsweise 5-50 µm, besonders bevorzugt 10-30 µm.

Der erfindungsgemäße Mehrschichtfilm eignet sich insbesondere als transmucosale Arzneimittelform. Diese Verwendung ist deshalb ebenfalls Gegenstand der vorliegenden Erfindung.

Der erfindungsgemäße Mehrschichtfilm wird dadurch hergestellt, daß die Schichten übereinander auf einer vorzugsweise glatten Oberfläche aufgebaut werden, indem die filmbildenden Polymeren ggf. der Vernetzer und ggf. der Wirkstoff pro Schicht jeweils durch Versprühen und Trocknen in Teilschichten aufgebracht werden.

Vorzugsweise wird jede Schicht aus 0,1-10 µm dicken Teilschichten aufgebaut.

Die Trocknung erfolgt vorzugsweise simultan mit dem Versprühen.

Als Lösungen werden vorzugsweise wässrige Lösungen eingesetzt.

Die Versprühung der filmbildenden, wässrigen Polymerlösung und des als wässrige Lösung vorliegenden Vernetzers der Deck- bzw. wirkstoffhaltigen Schicht erfolgt vorzugsweise gleichzeitig, wobei sich die Polymerlösung und der Vernetzer nach dem Versprühen vermischen und in situ vernetzen.

Das Einbringen des Wirkstoffes in die wirkstoffhaltige Schicht erfolgt vorzugsweise dadurch, daß der/die Wirkstoff(e) in den wässrigen Lösungen des Vernetzers gelöst oder, falls erforderlich, in Form flüssiger oder fester Teilchen emulgiert bzw. suspendiert werden. Besonders vorteilhaft ist hierbei das Eintragen von Feststoffen in die Polymerlösung, die hierbei bis zum Versprühen als Suspensionsstabilisator dient.

Teilschichten der wirkstoffhaltigen Schicht mit unterschiedlichen Wirkstoffkonzentrationen und/oder unterschiedlichen Wirkstoffen lassen sich erzielen, indem die von den Düsen versprühte Lösung gewechselt wird. Lokale ggf. unterschiedliche Konzentrationen von Wirkstoffen innerhalb einer wirkstoffhaltigen Schicht werden hergestellt, indem die jeweiligen Düsen mit unterschiedlichen, ggf. unterschiedlich konzentrierten Lösungen betrieben werden.

Das Versprühen der jeweiligen zur Herstellung des erfindungsgemäßen Mehrschichtfilms benötigten Substanzen erfolgt mit Ein- Zwei- und/oder Zweistoffdüsen, deren Sprühkegel sich überlappen. Vorzugsweise bewegt sich die glatte Oberfläche, auf der der Mehrschichtfilm aufgebaut wird, unter den Sprühkegeln zyklisch hindurch.

Um den erfindungsgemäßen Mehrschichtfilm mit einer möglichst gleichmäßigen Dicke herzustellen, ist es vorteilhaft, wenn sich die Sprühkegel überlappen und sich die glatten Oberflächen, auf denen der Mehrschichtfilm aufgebaut wird, vorzugsweise unter den Düsen zyklisch hindurch bewegen.

Die große Variabilität des erfindungsgemäßen Verfahrens erlaubt es, den Schichtaufbau in umgekehrter Reihenfolge durchzuführen. So kann auch mit der Haftschicht als erste Grundlage für die darauffolgenden wirkstoffhaltigen- und Deckschichten auf der Trägeroberfläche begonnen werden.

Das Aufbringen von mehr als drei Schichten ist mit dem erfindungsgemäßen Verfahren ebenfalls ohne weiteres möglich. So kann beispielsweise eine zusätzliche wirkstofffreie Sperrschicht auf der Abgabeseite zu einer Freigabeverzögerung von sonst rasch freigesetzten Wirkstoffen dienen.

Der erfindungsgemäße Mehrschichtfilm läßt sich am besten auf einer Vorrichtung herstellen, die mindestens eine Sprühvorrichtung, einen Trockner und mindestens eine Platte aufweist, die zyklisch unter der Sprühvorrichtung hindurch bewegt wird.

Vorzugsweise weist die Vorrichtung mehrere Düsen auf, deren Sprühkegel sich überlappen.

Der erfindungsgemäße Mehrschichtfilm ist einfach herzustellen und für die Umwelt nicht belastend, weil wässrige Lösungen zum Einsatz kommen. Der Mehrschichtfilm haftet z.B. sehr gut auch über einen längeren Zeitraum an der Mundschleimhaut. Probanden, denen der erfindungsgemäße Mehrschichtfilm in die Mundhöhle eingesetzt wurde, haben ein kaum störendes Fremdkörpergefühl.

Mit dem erfindungsgemäßen Verfahren lassen sich Mehrschichtfilme mit einer beliebigen Schichtkombination einfach und mit einer sehr hohen Präzision herstellen.

Im folgenden wird ein Mehrschichtfilm und das Verfahren zu dessen Herstellung anhand von Beispielen erläutert, die jedoch nur als Information dienen und nicht dem beanspruchten Gegenstand entsprechen.

### Beispiel 1 :Mucoadhäsiver, wirkstofffreier Mehrschichtfilm

Für die Herstellung von 50 g Film mit einer Oberfläche von 3472 cm² entsprechend 771 Filmstücken zu 4.5 cm² werden eingesetzt:
a. Deckschicht: 835g 2 %ige Lösung aus 16.7g MHPC 100 und 819g Wasser; sowie 835g einer 0.5 %ige Lösung aus 4.17g Tannin und 831 g Wasser; resultierende Schichtdicke des Teilfilms 50 µm.
b. Potentiell zur Aufnahme von Wirkstoff geeignete Mittelschicht: 835g 2 %ige Lösung aus 16.7g MHPC 100 und 819g Wasser, sowie 835g einer 0.5 %igen Lösung aus 4.17g Tannin und 2.35 g Carbopol 934 P; resultierende Schichtdicke des Teilfilms 50 µm.
c. Haftschicht: 842g einer 1 %igen Lösung aus 1.67 g Metholose 90 SH 100, 6.67 g Carbopol 934 P und 833g Wasser; resultierende Schichtdicke 20µm.

Die wässrigen Lösungen der Polymeren werden durch Einrühren der Polymerpulver in gereinigtes Wasser, das auf 80° C erwärmt wurde und anschließendem Kaltrühren auf Raumtemperatur mittels Flügelrührer bereitet.
Die für Deck- und Mittelschicht erforderlichen Lösungen - Polymer und Vernetzungsmittel - werden von Schlauchpumpen mit einer Fördermenge von 2,5 g.min⁻¹ geleitet und bei Düsenöfifnungen von jeweils 0,5 mm mit einem Zerstäuberdruck von 1 bar auf Glasplatten im Abstand von ca. 10 cm gesprüht.

Die Düsen sind so justiert, daß sich die Kegel der Sprühstrahlen auf den Glasträgern überlappen. Zur homogenen Verteilung der Filmmasse werden die Glasträger mit gleichmäßiger Geschwindigkeit zyklisch unter dem Sprühstrahl bewegt, wie dies durch Fixierung der Glasträger auf der Trommel eines mit konstanter Geschwindigkeit rotierenden Pelletierteller gewährleistet wird. In einem Zyklus können bis zu 0,5 µm dicke Filmlagen aufgesprüht werden. Die Sprühgeschwindigkeit und die Anzahl der Zyklen bestimmen damit die Dicke einer Teilschicht.
Das Trocknen der gesprühten Filme erfolgt simultan durch Zufuhr von Trockenluft, die eine Temperatur von max. 60° C aufweist.

Die Herstellung der einzelnen Schichten erfolgt unmittelbar nacheinander durch Wechsel der Sprühlösungen.

Nach dem Trocknen läßt sich der Gesamtfilm leicht von den Glasträgern abnehmen. Er besitzt eine Dicke von 120 µm ± 4 µm.

Die mittleren Haftzeiten des Mehrschichtfilms in der Mundhöhle beträgt:

| | |
|---|---|
| Gingival | 470 min |
| Palatal | 210 min |
| Buccal | 120 min |

Zum Ende der Applikation können die Mehrschichtfilme sehr leicht als gequollene Scheiben mit weißlicher Färbung abgenommen werden.

### Beispiel 2: Mehrschichtfilm mit 1% Prednisolon

Für die Herstellung von 50 g Film mit einer Oberfläche von 3439 cm², entsprechend 764 Filmstücken von jeweils 4.5 cm² werden eingesetzt:
a. Deckschicht: 825 g 2 %ige Lösung aus 16.5 g MHPC 100 und 809 g Wasser, sowie 825 g 0.5%ige Lösung aus 4.1 g Tannin und 821 g Wasser; resultierende Schichtdicke 50 µm.
b. Wirkstoffhaltige Schicht: 825 g 2%ige Lösung aus 16.5 g MHPC 100, 0.48 g feindispersem Prednisolon und 808 g Wasser, sowie 825 g Lösung aus 4.1 g Tannin und 821 g Wasser; resultierende Schichtdicke 50 µm.
c. Haftschicht: 833 g 1 %ige Lösung aus 6.67 g Carbopol 934 P, 1.67 g MHPC 100 und 825 g Wasser; resultierende Schichtdicke 20 µm.

Die Herstellung erfolgt wie in Beispiel 1 beschrieben. Zum Aufbau der wirkstoffhaltigen Schicht wird, abweichend von Beispeil1 eine 2%ige MHPC Lösung eingesetzt, in der mikronisiertes Prednisolon durch Einrühren während der Abkühlphase der Lösung suspendiert wird.

Die Gesamtfilmdicke beträgt nach dem Trocknen 120µm ± 4 µm

### Beispiel 3: Mehrschichtfilm mit 1.3% Prednisolon und verstärkter Deckschicht

Für die Herstellung von 50 g Film mit einer Gesamtfläche von 3069 cm² entsprechend 682 Filmstücken von 4.5 cm² werden eingesetzt:
a. Deckschicht: 937 g 2 %ige Lösung aus 18.73 g MHPC 100 und 918 g Wasser, sowie 937 g 0.5%ige Lösung aus 4.72 g Tannin und 933 g Wasser; resultierende Schichtdicke 64 µm.
b. Wirkstoffhaltige Schicht: 737 g 2 %ige Lösung aus 14.73 g MHPC 100, 0.65g feindispersem Prednisolon und 722 g Wasser, sowie 737 g 0.5 %ige Lösung aus 3.68 g Tannin und 734 g Wasser; resultierende Schichtdicke 50 µm.
c. Haftschicht: 736 g 1%ige Lösung aus 5.89 g Carbopol 934 P, 1.47 g MHPC 100 und 729 g Wasser; resultierende Schichtdicke 20 µm.

Die Herstellung erfolgt wie in Beispiel 1 bzw. Beispiel 2 beschrieben. Die Filmdicke des Gesamtfilms beträgt nach dem Trocknen 134 µm ± 5 µm.

### Beispiel 4:Mehrschichtfilm mit 4.6% Prednisolon

Für die Herstellung von 50 g Film mit einer Gesamtfläche von 3314 cm² entsprechend 736 Filmstücken von 4.5 cm² werden eingesetzt:
a. Deckschicht: 796 g 2%ige Lösung aus 15.91 g HPMC 100 und 780 g Wasser, sowie 796 g 0.5%ige Lösung aus 4 g Tannin und 792 g Wasser; resultierende Schichtdicke 50 µm.
b. Mittelschicht: 796 g 0.5%ige Lösung aus 15.91 g MHPC 100, 2.29 g feindispersem Prednisolon und 778 g Wasser, sowie 796 g 0.5%ige Lösung aus 4 g Tannin und 792 g Wasser; resultierende Schichtdicke 50 µm.
c. Haftschicht: 795 g 1%ige Lösung aus 6.3 g Carbopol 934 P, 1.59 g MHPC 100 und 787 g Wasser; resultierende Schichtdicke 20 µm.

Die Herstellung erfolgt wie im Beispiel 2. beschrieben. Die Schichtdicke des Gesamtfilms beträgt 120 µm ± 5 µm.

Die beschriebenen Mehrschichtfilme mit Carbopol 934 P und Metholose 90 SH 100 als Haftschicht weisen im in vivo-Test eine unerwartet gute Haftfähigkeit an unterschiedlichen Applikationsstellen in der Mundhöhle auf, wobei die sensorischen Eindrücke von Probanden sich auf ein kaum störendes Fremdkörpergefühl.

## Patentansprüche

1. Mehrschichtfilm aus filmbildenden Polymeren mit einer Deckschicht, mindestens einer wirkstoffhaltigen Schicht und einer Haftschicht, wobei die wirkstoffhaltige Schicht aus in situ vernetzten, hydrophilen Polymeren besteht, **dadurch gekennzeichnet, daß** wenigstens eine wirkstoffhaltige Schicht bezüglich des Wirkstoffes einen horizontalen und/oder vertikalen Gradienten aufweist oder der Wirkstoff in bestimmten horizontalen und/oder vertikalen Segmenten der wirkstoffhaltigen Schicht konzentriert ist.

2. Mehrschichtfilm gemäß Anspruch 1, **dadurch gekennzeichnet, daß** auch die Deckschicht aus in situ vernetzten, hydrophilen Polymeren besteht.

3. Mehrschichtfilm gemäß Anspruch 1 oder 2, **dadurch gekennzeichnet, daß** die Deckschicht und die wirkstoffhaltige Schicht jeweils zwischen 30 und 100 µm dick sind.

4. Mehrschichtfilm gemäß einem der Ansprüche 1-3, **dadurch gekennzeichnet, daß** als hydrophile Polymere Celluloseether, vorzugsweise Hydroxyethylcellulose, Methylcellulose, besonders bevorzugt Methylhydroxypropylcellulose eingesetzt wurden, die vorzugsweise mit phenolischen Substanzen, besonders bevorzugt mit Tannin vernetzt wurden.

5. Mehrschichtfilm gemäß einem der Ansprüche 1-3, **dadurch gekennzeichnet, daß** als hydrophile Polymere anionische Polymere, vorzugsweise Natriumcarboxymethylcellulose, Polyacrylate oder Carragenate, besonders bevorzugt Natriumalginat eingesetzt wurden, die vorzugsweise mit anorganischen Ionen, besonders bevorzugt Kalciumionen, oder vorzugsweise mit Polykationen, besonders bevorzugt Chitosan vernetzt wurden.

6. Mehrschichtfilm gemäß einem der Ansprüche 1-5, **dadurch gekennzeichnet, daß** die Deckschicht Hilfsstoffe, vorzugsweise Farbstoffpigmente und/oder Geschmacksstoffe enthält.

7. Mehrschichtfilm nach einem der Ansprüche 1-6, **dadurch gekennzeichnet, daß** die Haftschicht Methylhydroxypropylcellulose und Polyacrylsäure enthält.

8. Mehrschichtfilm nach einem der Ansprüche 1-7, **dadurch gekennzeichnet, daß** er mehrere Wirkstoffe aufweist.

9. Mehrschichtfilm gemäß Anspruch 8, **dadurch gekennzeichnet, daß** jede wirkstoffhaltige Schicht jeweils mindestens einen Wirkstoff enthält.

10. Mehrschichtfilm nach einem der Ansprüche 1-9, **dadurch gekennzeichnet, daß** die Wirkstoffe Duftstoffe, Pflanzenschutzmittel, pharmazeutische Wirkstoffe, Vitamine, Nährstoffe und/oder Düngemittel sind.

11. Verwendung eines Mehrschichtfilms gemäß einem der Ansprüche 1-10 zur Herstellung einer transmucosalen Arzneimittelform.

12. Verfahren zur Herstellung eines Mehrschichtfilms gemäß einem der Ansprüche 1-10, **dadurch gekennzeichnet, daß** die Schichten übereinander auf einer vorzugsweise glatten Oberfläche aufgebaut werden, indem das filmbildende Polymer, der Vernetzer und der Wirkstoff oder die Wirkstoffe pro wirkstoffhaltiger Schicht in Teilschichten jeweils durch Versprühen der für die Herstellung der Schicht erforderlichen Lösungen übereinander auf die glatte Oberfläche und Trocknen aufgebracht werden.

13. Verfahren gemäß Anspruch 12, **dadurch gekennzeichnet, daß** zur Herstellung der Deck- und der wirkstoffhaltigen Schicht(en) eine Lösung der filmbildenden Polymeren und das Vernetzungsmittel gleichzeitig versprüht werden.

14. Verfahren gemäß dem Anspruch 12 oder 13, **dadurch gekennzeichnet, daß** das Versprühen mit mindestens einer Ein-, Zwei- oder Dreistoffdüse erfolgt.

15. Verfahren gemäß einem der Ansprüche 12-14, **dadurch gekennzeichnet, daß** der Wirkstoff in einer wässrigen Lösung der Vemetzungskomponente vorzugsweise gelöst, emulgiert oder suspendiert vorliegt.

16. Verfahren gemäß einem der Ansprüche 12 - 15, **dadurch gekennzeichnet, daß** die für die Herstellung einer wirkstoffhaltigen Schicht erforderlichen Lösungen übereinander auf eine Platte gesprüht werden, wobei sich die Sprühkegel überlappen und die Platte sich zyklisch unter den Sprühkegeln durchbewegt.

## Claims

1. Multi-layer film consisting of film-forming polymers comprising a cover layer, at least one layer containing an active substance, and an adhesive layer, wherein the active substance-containing layer consists of hydrophilic polymers crosslinked *in situ,* **characterised in that** at least one active substance-containing layer has a horizontal and/or vertical gradient with respect to the active substance, or the active substance is concentrated in specific horizontal ahd/or vertical segments of the active substance-containing layer.

2. Multi-layer film according to claim 1, **characterised in that** the cover layer also consists of hydrophilic polymers crosslinked *in situ.*

3. Multi-layer film according to either claim 1 or claim 2, **characterised in that** the cover layer and the active substance-containing layer are each between 30 and 100 µm thick.

4. Multi-layer film according to any one of claims 1 to 3, **characterised in that** said hydrophilic polymers are cellulose ethers, preferably hydroxyethylcellulose, methylcellulose, particularly preferably methylhydroxypropylcellulose, which have preferably been crosslinked with phenolic substances, particularly preferably with tannin.

5. Multi-layer film according to any one of claims 1 to 3, **characterised in that** said hydrophilic polymers are anionic polymers, preferably sodium carboxymethylcellulose, polyacrylates or carragenates, particularly preferably sodium alginate, which have preferably been crosslinked with inorganic ions, particularly preferably calcium ions, or preferably with polycations, particularly preferably chitosan.

6. Multi-layer film according to any one of claims 1 to 5, **characterised in that** the cover layer contains auxiliary substances, preferably dye pigments and/or flavouring agents.

7. Multi-layer film according to any one of claims 1 to 6, **characterised in that** the adhesive layer contains methylhydroxypropylcellulose and polyacrylic acid.

8. Multi-layer film according to any one of claims 1 to 7, **characterised in that** it comprises a plurality of active substances.

9. Multi-layer film according to claim 8, **characterised in that** each active substance-containing layer contains at least one active substance.

10. Multi-layer film according to any one of claims 1 to 9, **characterised in that** the active substances are aromas, plant protection agents, pharmaceutical active substances, vitamins, nutrients and/or fertilisers.

11. Use of a multi-layer film according to any one of claims 1 to 10 for the preparation of a transmucosal pharmaceutical form.

12. Process for the preparation of a multi-layer film according to any one of claims 1 to 10, **characterised in that** the layers are successively built up on a preferably smooth surface **in that**, for each active substance-containing layer, the film-forming polymer, the crosslinking agent and the active substance or the active substances are in each case successively applied in partial layers to the smooth surface by spraying the solutions required for the preparation of the layer, followed by drying.

13. Process according to claim 12, **characterised in that** the cover layer and the active substance-containing layer(s) are prepared by simultaneously spraying a solution of the film-forming polymers and the crosslinking agent.

14. Process according to either claim 12 or claim 13, **characterised in that** the spraying is carried out using at least one single-component, two-component or three-component nozzle.

15. Process according to any one of claims 12 to 14, **characterised in that** the active substance is preferably dissolved, emulsified or suspended in an aqueous solution of the crosslinking component.

16. Process according to any one of claims 12 to 15, **characterised in that** the solutions required for the preparation of an active substance-containing layer are successively sprayed onto a plate, wherein the spray cones overlap and the plate moves cyclically underneath the spray cones.

## Revendications

1. Pellicule multicouche en polymères filmogènes comprenant une couche de revêtement, au moins une couche contenant une substance active et une couche adhésive, la couche contenant une substance active étant constituée de polymères hydrophiles réticulés in situ, **caractérisée en ce qu'**au moins une couche contenant une substance active présente un gradient horizontal et/ou vertical par rapport à la substance active ou bien cette dernière est concentrée dans certains segments horizontaux et/ou verticaux de la couche qui la contient.

2. Pellicule multicouche suivant la revendication 1, **caractérisée en ce que** la couche de revêtement est aussi constituée de polymères hydrophiles réticulés in situ.

3. Pellicule multicouche suivant la revendication 1 ou 2, **caractérisée en ce que** la couche de revêtement et la couche contenant la substance active ont chacune une épaisseur comprise entre 30 et 100 µm.

4. Pellicule multicouche suivant l'une des revendications 1 à 3, **caractérisée en ce que** les polymères hydrophiles qui ont été utilisés sont des éthers de cellulose, avantageusement l'hydroxyéthylcellulose, la méthylcellulose, notamment la méthylhydroxypropylcellulose, qui ont été avantageusement réticulés avec des substances phénoliques, notamment avec un tanin.

5. Pellicule multicouche suivant l'une des revendications 1 à 3, **caractérisée en ce que** les polymères hydrophiles qui ont été utilisés sont des polymères anioniques, avantageusement la carboxyméthylcellulose sodique, des polyacrylates ou des carraghénates, notamment l'alginate de sodium, qui ont été réticulés avantageusement avec des ions inorganiques, notamment des ions calcium, ou avantageusement avec des polycations, notamment un chitosane.

6. Pellicule multicouche suivant l'une des revendications 1 à 5, **caractérisée en ce que** la couche de revêtement contient des substances auxiliaires, avantageusement des pigments colorés et/ou des substances modifiant le goût.

7. Pellicule multicouche suivant l'une des revendications 1 à 6, **caractérisée en ce que** la couche adhésive contient de la méthylhydroxypropylcellulose et un polymère d'acide acrylique.

8. Pellicule multicouche suivant l'une des revendications 1 à 7, **caractérisée en ce qu'**elle contient plusieurs substances actives.

9. Pellicule multicouche suivant la revendication 8, **caractérisée en ce que** chaque couche chargée de substance active contient dans chaque cas au moins une substance active.

10. Pellicule multicouche suivant l'une des revendications 1 à 9, **caractérisée en ce que** les substances actives sont des parfums, des agents phytosanitaires, des substances pharmaceutiques actives, des vitamines, des substances nutritives et/ou des engrais.

11. Utilisation d'une pellicule multicouche suivant l'une des revendications 1 à 10 pour la préparation d'une forme médicamenteuse agissant à travers des muqueuses.

12. Procédé de production d'une pellicule multicouche suivant l'une des revendications 1 à 10, **caractérisé en ce qu'**on édifie les couches en les superposant sur une surface de préférence lisse, en appliquant par superposition sur la surface lisse le polymère filmogène, l'agent de réticulation et la ou les substances actives, par couche chargée de substances actives, en couches partielles, dans chaque cas par pulvérisation des solutions nécessaires pour la production de la couche, et en séchant.

13. Procédé suivant la revendication 12, **caractérisé en ce qu'**une solution des polymères filmogènes et l'agent de réticulation sont pulvérisés en même temps pour la production de la couche de revêtement et de la couche ou des couches chargées de substances actives.

14. Procédé suivant la revendication 12 ou 13, **caractérisé en ce que** la pulvérisation est effectuée avec au moins une buse à une, deux ou trois substances.

15. Procédé suivant l'une des revendications 12 à 14, **caractérisé en ce que** la substance active est avantageusement mise en solution, en émulsion ou en suspension dans une solution aqueuse du composant de réticulation.

16. Procédé suivant l'une des revendications 12 à 15, **caractérisé en ce que** les solutions nécessaires pour produire une couche chargée de substance active sont pulvérisées en superposition sur une plaque, les cônes de pulvérisation se recouvrant et la plaque se déplaçant de façon cyclique en passant sous les cônes de pulvérisation.
